(19) Europäisches Patentamt
European Patent Office
Office européen des brevets

(11) EP 0 495 971 B1

(12) **FASCICULE DE BREVET EUROPEEN**

(45) Date de publication et mention de la délivrance du brevet:
**19.11.1997 Bulletin 1997/47**

(21) Numéro de dépôt: **91915653.9**

(22) Date de dépôt: **09.08.1991**

(51) Int Cl.[6]: **G01N 33/94**, G01N 33/86, G01N 33/569, G01N 33/50, G01N 33/564

(86) Numéro de dépôt international:
**PCT/FR91/00659**

(87) Numéro de publication internationale:
**WO 92/02823 (20.02.1992 Gazette 1992/05)**

(54) **DETERMINATION DES THROMBOPENIES**
NACHWEIS VON THROMBOPENIES
THROMBOCYTOPENIA DETERMINATION

(84) Etats contractants désignés:
**AT BE CH DE DK ES FR GB GR IT LI LU NL SE**

(30) Priorité: **09.08.1990 FR 9010190**

(43) Date de publication de la demande:
**29.07.1992 Bulletin 1992/31**

(73) Titulaire: **DIAGNOSTICA STAGO (société anonyme)**
**F-92602 Asnières (FR)**

(72) Inventeur: **AMIRAL, Jean**
**F-95130 Franconville (FR)**

(74) Mandataire: **Clisci, Serge et al**
**S.A. FEDIT-LORIOT**
**CONSEILS EN PROPRIETE INDUSTRIELLE**
**38, avenue Hoche**
**75008 Paris (FR)**

(56) Documents cités:
**EP-A- 0 165 681**

- **THROMBOSIS RESEARCH vol. SUPPL, no. VI, 1986, NEW YORK NY USA page 170; J. AMIRAL ET AL: 'Elisa evaluation of beta thromboglobulin and platelet factor 4' Voir résumé nr. 335 en entier.**
- **BLOOD vol. 66, no. 5, 5 Décembre 1985, WASHINGTON DC USA pages 1176 - 1181; D. M. LYNCH ET AL.: 'Heparin-associated thrombocytopenia: antibody binding specificity to platelet antigens.'**
- **THE JOURNAL OF LABORATORY AND CLINICAL MEDICINE vol. 91, no. 1, 1 Janvier 1978, ST. LOUIS MO USA pages 167 - 175; D. GREEN ET AL: 'Heparin immune thrombocytopenia: evidence for a heparin-platelet complex as the antigenic determinant.' En entier.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 495 971 B1

**Description**

**DOMAINE DE L'INVENTION**

La présente invention concerne un procédé mettant en oeuvre une substance antigénique particulière pour la détermination des thrombopénies (également dénommées thrombocytopénies) induites par l'héparine.

**ART ANTERIEUR**

On sait, notamment de l'article de M.C. BERNDT et al, Blood Reviews 1, pages 111-118, (1987), que les thrombopénies peuvent être induites par de nombreux médicaments, et que les plus fréquentes sont provoquées par la quinine/quinidine et surtout l'héparine.

On sait que l'on administre l'héparine, en tant que moyen anticoagulant, aux personnes hospitalisées pour prévenir tout risque de thrombus veineux ou artériel. Statistiquement, on sait que (i) au moins 50 % des patients hospitalisés reçoivent de l'héparine par injection, et que (ii) 1 à 5 % des patients soumis à une héparino-thérapie sont atteints de thrombopénies pouvant se développer de façon particulièrement sévère entre le 5ème et le 15ème jours de l'héparino-thérapie. Voir à cet effet les articles de

- J.G. KELTON et al., Blood 72 (No 2), pages 925-930, (1988) ;
- B.H. CHONG et al., British Journal of Haematology 49, pages 531-540, (1981) ;
- B.H. CHONG, Blood Reviews 2, pages 108-114, (1986) ;
- D. SHERIDAN et al., Blood 67 (No 1), pages 27-30, (1986) ;
- Y. GRUEL, Sang Thrombose Vaisseaux, 1 (No 4), pages 233-236, (1989) ;
- M. SAMAMA et al., Journal des Maladies Vasculaires 7, pages 237-242, (1982) ; et
- J. CONARD et al., intitulé : "Les thrombopénies à l'héparine" et publié dans l'ouvrage "Progrès en Hématologie 4, Les Plaquettes Sanguines", pages 107-118, Doin Editeurs, Paris (1983).

Chez les patients bénéficiant d'une héparino-thérapie et développant des thrombopénies, on connaît deux formes :

- les thrombopénies dites légères ou modérées qui sont asymptomatiques, et
- les thrombopénies dites sévères fréquemment accompagnées de complications thromboemboliques artérielles ou veineuses résultant de l'apparition d'anticorps dirigés contre les plaquettes en présence d'héparine.

Cette apparition d'anticorps est notamment illustrée par l'article de D.M. LYNCH et al., Blood 66 (No 5), pages 1176-1181, (1985) et par les articles précités de M.C. BERNDT et al., J.G. KELTON et al., B.H. CHONG et al., B.H. CHONG, D. SHERIDAN et al., et J. CONARD et al. (voir en particulier le mécanisme des thrombopénies induites par l'héparine présenté dans le tableau III, page 115 dudit article de J. CONARD et al.).

Le problème de la prophylaxie des accidents thrombopéniques n'est pas encore résolu. Il existe néanmoins le besoin d'une méthode de dosage rapide et fiable permettant d'apprécier le risque de développement de thrombopénies afin de stopper suffisamment tôt l'administration du médicament inducteur (ou drogue inductrice).

A l'heure actuelle, les méthodes utilisées pour le diagnostic des thrombopénies sont celles qui comportent

(1) la recherche de l'absence d'une étiologie différente des thrombopénies (infections, autres thérapies, etc...) qui est longue et fastidieuse,
(2) la numération des plaquettes sanguines avant, pendant et après traitement, qui est longue et peu spécifique, ou
(3) les tests biologiques (énoncés ci-après) recherchant l'apparition d'anticorps dirigés contre les plaquettes en présence du médicament inducteur ou de la drogue inductrice.

Il se trouve que les examens biologiques les plus fréquemment utilisés sont les tests d'agrégation plaquettaire, qui nécessitent un appareillage adapté et mettent en oeuvre des modalités opératoires longues et manquant dans certains cas de sensibilité.

Les autres méthodes visées au point (3) ci-dessus, qui ont été décrites (voir en particulier les articles de J.G. KELTON et al., B.H. CHONG et al., B.H. CHONG, D. SHERIDAN et al. et Y. GRUEL précités), mettent en oeuvre l'étude de la fixation plaquettaire des IgG sériques, la libération de la sérotonine $^{14}C$-radiomarquée à deux concentrations différentes d'héparine, la disponibilité du facteur plaquettaire 3, la fixation du complément, l'inhibition de la lyse du complément, et l'agglutination d'hématies (notamment de mouton) sensibilisées. Il se trouve que ces méthodes d'essais biologiques sont ou bien peu sensibles ou peu fiables, ou bien, si elles sont sensibles, longues à réaliser.

Il en est de même en ce qui concerne l'enseignement de l'article de D.M. LYNCH et al. précité et de EP-A-0 165 681.

Selon ledit article de D.M. LYNCH et al., on se propose de détecter, sur des plaques ELISA contenant (i) des plaquettes humaines fixées sur la paroi, (ii) du sérum de patient ayant une thrombopénie associée à l'héparine et (iii) de l'héparine, la présence d'Ig pouvant se lier aux plaquettes et dépendant de l'héparine [i.e. "heparin-dependent serum platelet-bindable immunoglobulin" (S-PBIg)] au moyen d'un anticorps anti(Ig) lié à la peroxydase. Cette technique implique le traitement des plaquettes avec le sérum du patient.

Selon EP-A-0 165 681, on propose un procédé pour détecter une thrombopénie due à une drogue inductrice (ici Qn/Qnd) suivant lequel on traite des plaquettes sanguines avec le sérum ou le plasma d'un patient susceptible d'être thrombopénique et la drogue inductrice, incube à 21-24°C pendant 15-25 minutes, lave les plaquettes incubées avec une solution contenant la drogue inductrice, met en contact et incube les plaquettes résultantes (mises en suspension) avec un support solide comportant une protéine de liaison spécifique, décante les plaquettes non liées et quantifie les plaquettes liées, au moyen d'un enzyme et de son substrat.

Par ailleurs, on connaît de M.B. ZUCKER et al., Proc. Natl. Acad. Sci. USA, 86, pages 7571-7574, (1989) et de EP-A-0 378 364 (date de publication : 18 juillet 1990) la structure du pF4 et l'obtention du pF4 recombinant par génie génétique.

**BUT DE L'INVENTION**

Selon l'invention, on propose une nouvelle solution technique pour la détermination des thrombopénies induites par une drogue inductrice (Z) qui est une substance héparinique (Hep). Cette nouvelle solution technique repose sur la détection, (i) au moyen d'une substance antigénique (Ag) spécifique, (ii) d'un matériau immunologique anticorps contenu dans le plasma du sujet à tester et choisi parmi l'ensemble constitué par les anticorps anti(Y) (où Y est notamment Z ou les complexes Z-Ag) dirigés notamment contre la drogue inductrice Z et ses complexes avec Ag.

Eu égard aux résultats des travaux entrepris par la Demanderesse, il a été trouvé que, chez les animaux à sang chaud, notamment l'homme et les mammifères, l'administration d'une drogue inductrice de thrombopénie produit des anticorps anti(Z), anti(Z-plaquette), anti(Z-Ag) et anti(Z-Ag-plaquette) conduisant à une agrégation ou une activation des plaquettes sanguines provoquant la thrombopénie.

La nouvelle solution technique selon l'invention pour la détermination des thrombopénies met en oeuvre une substance antigénique particulière, obtenue notamment par le clivage ou la lyse de plaquettes sanguines et ayant une forte affinité vis-à-vis de la drogue inductrice.

Cette nouvelle solution technique offre l'avantage, à la différence des solutions antérieurement connues, d'être fiable, très sensible et rapide de mise en oeuvre.

Le résultat de la réaction antigène-anticorps peut être détecté, signalé ou amplifié selon une méthode connue en soi (notamment par agglutination, EIA, RIA ou FIA).

**OBJET DE L'INVENTION**

Suivant l'invention, on préconise un procédé pour la détermination des thrombopénies induites par une substance héparinique (Hep) en tant que drogue inductrice, ledit procédé étant caractérisé en ce qu'il comprend les étapes consistant :

(1°) à faire réagir (a) une substance antigénique (Ag ou $Ag_1$), la substance antigénique Ag étant choisie parmi l'ensemble constitué par

- (A) le facteur plaquettaire 4 (pF4),
- (B) les fractions contenant le pF4,
- (C) les fractions contenant au moins une substance éluée en même temps que le pF4,
- (D) le pF4 recombinant et ses variants,
- (E) les peptides synthétiques reprenant tout ou une partie de la séquence des aminoacides du pF4,
- (F) le protéoglycan,
- (G) les complexes protéoglycan-pF4, et
- (H) leurs mélanges,

et la substance antigénique $Ag_1$ étant un complexe Hep-Ag de ladite substance héparinique Hep avec ladite substance antigénique Ag, avec (b) le plasma d'un patient susceptible de contenir un matériau anticorps anti(Hep-Ag), qui est généré dans l'organisme après administration de Hep, en cas de thrombopénie héparinique, et qui reconnaît Ag, selon la réaction

$$Ag + anti(Hep\text{-}Ag) \rightarrow Ag\text{-}anti(Hep\text{-}Ag),$$

ou

$$(Hep\text{-}Ag) + anti(Hep\text{-}Ag) \rightarrow (Hep\text{-}Ag)\text{-}anti(Hep\text{-}Ag) ;$$

puis
(2°) à révéler le complexe produit résultant de ladite réaction.

Selon l'invention on propose enfin des nécessaires, trousses ou kits de dosage pour la détermination des thrombopénies, qui comprennent au moins un échantillon de ladite substance antigénique précitée ou l'un de ses complexes, et, le cas échéant, des milieux de dilution appropriés et/ou d'autres réactifs.

## ABREVIATIONS

Dans ce qui suit, par commodité les abréviations suivantes ont été utilisées.

| | |
|---|---|
| Ag | désigne la substance antigénique selon l'invention ayant une forte affinité vis-à-vis de la drogue inductrice Z, c'est-à-dire ici la substance héparinique (Hep) ; |
| $Ag_1$ | désigne le complexe Hep-Ag ; |
| anti(Z) | désigne un anticorps généré contre la drogue inductrice (Z) ; |
| anti(X) | désigne tout anticorps généré contre la substance X, ainsi anti(Z), anti(Ig), anti(IgA), anti(IgG) et respectivement anti(IgM) désignent tout anticorps généré contre la drogue inductrice Z, les immuno-globulines Ig, les IgA, les IgG et respectivement les IgM; |
| EIA | désigne un essai enzymo-immunologique (en anglais : "enzyme immunoassay"); |
| ELISA | abréviation de l'expression anglaise "enzyme-linked immunosorbent assay", désigne une technique EIA particulière ; |
| F(ab) | désigne un premier fragment d'un anticorps obtenu par clivage dudit anticorps par la papaine; |
| $F(ab')_2$ | désigne un second fragment d'un anticorps obtenu par clivage dudit anticorps par la pepsine; |
| Fc | désigne tout fragment d'anticorps, séparé du fragment F(ab) par clivage au moyen de la papaine ou séparé du fragment $F(ab')_2$ par clivage au moyen de la pepsine ; lesdits fragments Fc sont homologues mais légèrement structurellement différents selon qu'ils ont été clivés par la papaine ou la pepsine (la structure et le mode d'obtention du F(ab), du $F(ab')_2$ et des Fc sont illustrés dans la demande de brevet français No 89 04 589 déposée le 7 avril 1989) ; |
| FIA | désigne un essai fluoroimmunologique (en anglais : "fluorescent immunoassay") |
| FR | désigne le Facteur Rhumatoïde ; |
| Hep | désigne une substance héparinique, à savoir l'héparine, ses dérivés ou ses analogues (y compris leurs complexes avec les plaquettes) ; |
| HRGP | désigne une glycoprotéine riche en histidine (en anglais : "histidine-rich glycoprotein") ; |
| Ig | désigne toute immunoglobuline ; |
| IgA | désigne toute immunoglobuline A ; |
| IgG | désigne toute immunoglobuline G ; |
| IgM | désigne toute immunoglobuline M ; |
| LA-pF4 | désigne un précurseur de βTG (en anglais : "low-affinity pF4") ; |
| OD | désigne la densité optique (mesurée notamment à une longueur d'onde de 492 nm) ; |
| OPD | désigne l'ortho-phénylènediamine ; |
| PBP | désigne le précurseur du LA-pF4, à savoir la protéine basique de plaquette (en anglais : "platelet basic protein") ; |
| pF3 | désigne le facteur plaquettaire 3 ; |
| pF4 | désigne le facteur plaquettaire 4 ; |
| PM | désigne le poids moléculaire ; |
| POD | désigne la peroxydase ; |
| Qn/Qnd | désigne la quinine, la quinidine, leurs mélanges, les dérivés ou analogues correspondants |
| R* | désigne un moyen de marquage ; |
| RIA | désigne un essai radio-immunologique (en anglais : "radio immunoassay") |
| RT | désigne la température ambiante (15-25°C) ; |

βTG désigne la béta-thromboglobuline et résulte de la dégradation ou du clivage partiel de LA-pF4 ;
Y désigne la drogue inductrice Z, les produits la contenant et ses dérivés et/ou analogues;
Z désigne la drogue inductrice de thrombopénies, c'est-à-dire ici Hep ;

## DESCRIPTION DETAILLEE DE L'INVENTION

La présente invention concerne la détermination de thrombopénies d'origine immune.

Le matériau antigénique Ag est constitué par des fragments plaquettaires (y compris les parois), des fractions intraplaquettaires libérables des plaquettes sanguines par clivage ou lyse, et éventuellement des substances plasmatiques ou sanguines. La substance antigéniques $Ag_1$, qui est un complexe Hep-Ag comprend les complexes desdits fragments plaquettaires, desdites fractions intraplaquettaires et desdites substances plasmatiques ou sanguines.

Par l'expression "ayant une forte affinité vis-à-vis de la drogue inductrice Z" appliquée à la substance antigénique, on entend le fait que la substance antigénique Ag réagit aisément avec la drogue inductrice Z, les dérivés de Z, les analogues de Z et les produits contenant Z.

Eu égard à l'affinité de Ag pour la drogue inductrice Z, les dérivés de Z (notamment les métabolites), les analogues de Z (notamment les héparinoïdes) et les produits contenant Z, on obtient l'ensemble des complexes inclus dans la définition de $Ag_1$ à partir de Ag.

On préconise en particulier pour la détermination des thrombopénies les plus fréquentes, qui sont induites par Hep, l'utilisation d'une substance antigénique Ag ou Hep-Ag ayant une forte affinité vis-à-vis de Hep et qui est destinée à réagir avec un matériau anticorps anti-héparine contenu dans le plasma à tester et dirigé contre Hep, les complexes Hep-plaquette, Hep-substance antigénique et/ou Hep-substance antigénique-plaquette,

où

Hep représente l'héparine, les composés qui dérivent de l'héparine, les composés analogues à l'héparine ou leurs mélanges,

ledit matériau anticorps produisant une agrégation ou une activation des plaquettes sanguines provoquant la thrombopénie.

Compte tenu des définitions données ci-dessus, la drogue inductrice Hep englobe tout produit choisi parmi l'ensemble constitué par

- l'héparine proprement dite ayant un PM moyen de 6 000-30 000 daltons et un pouvoir rotatoire $[\alpha]_D^{20}$ d'environ + 55° ;
- les dérivés de l'héparine, notamment les héparinates métalliques ($Ca^{2+}$, $Li^+$,$Na^+$, $Mg^{2+}$, etc...) et les fragments d'héparine;
- les analogues de l'héparine, notamment les héparinoïdes (héparamine et ses sels, les chondroitines et leurs sels, etc...), et les héparines ayant un poids moléculaire moyen inférieur à 6 000 daltons ;
- les substances contenant l'héparine, ses dérivés et analogues, notamment les complexes de l'héparine et de ses dits dérivés et analogues ; et
- leurs mélanges.

Comme indiqué plus haut, le procédé selon l'invention pour la détermination des thrombopénies induites par une drogue inductrice (Z) comprend la réaction (A) d'une substance antigénique Ag ou $Ag_1$ avec (B) un matériau anticorps anti(Y), où anti(Y) représente un matériau anticorps dirigé contre (a) Z, (b) les dérivés de Z, (c) les analogues de Z, (d) les produits contenant Z, ses dérivés ou ses analogues, ou (e) leurs mélanges.

De façon préférée, le matériau anticorps anti(Y) est un matériau anticorps anti(Hep-Ag) ; en pratique on utilisera le plasma du patient à tester comme source de matériau anti(Y) qui contient en général les anti(Z) et surtout les anti (Z-Ag), à savoir plus précisément le plasma d'un patient susceptible de contenir un matériau anticorps anti(Hep-Ag), qui est généré dans l'organisme après administration de Hep, en cas de thrombopénie héparinique, et qui reconnaît Ag, selon la réaction

$$Ag + anti(Hep\text{-}Ag) \rightarrow Ag\text{-}anti(Hep\text{-}Ag),$$

ou

$$(Hep\text{-}Ag) + anti(Hep\text{-}Ag) \rightarrow (Hep\text{-}Ag)\text{-}anti(Hep\text{-}Ag).$$

On a par ailleurs, constaté que lorsqu'on part d'une substance antigénique $Ag_1$ (à savoir ici Hep-Ag), on améliore

la sensibilité du dosage en ajoutant au dit complexe une quantité appropriée de la drogue inductrice Hep.

Pour la détermination des thrombopénies induites par l'héparine, on préconise selon l'invention un procédé, qui comporte la réaction d'un antigène (1)

spécifique de l'auto-anticorps (2) généré par la drogue inductrice après administration de celle-ci,

avec ledit auto-anticorps (2), ledit procédé étant caractérisé en ce que ledit antigène (1) est une substance antigénique Ag ou Hep-Ag.

Dans ce procédé relatif à la détermination des thrombopénies induites par l'héparine, l'auto-anticorps (2) est comme indiqué ci-dessus l'anticorps anti(Hep-Ag).

Pour la détermination des thrombopénies induites par l'héparine ladite substance antigénique Ag sera choisie parmi l'ensemble constitué par

(A) le facteur plaquettaire 4 (pF4),
(B) les fractions contenant le pF4,
(C) les fractions contenant au moins une substance éluée en même temps que le pF4,
(D) le pF4 recombinant et ses variants,
(E) les peptides synthétiques reprenant tout ou une partie de la séquence des aminoacides du pF4 (en particulier les peptides carboxy-terminaux 1-13 ou 13-24),
(F) le protéoglycan,
(G) les complexes protéoglycan-pF4, et
(H) leurs mélanges,

Parmi les fractions contenant le pF4 visées en tant que moyen (B) ci-dessus, on peut mentionner notamment les polymères du pF4 comprenant plusieurs chaînes parallèles du pF4 monomère.

Parmi les fractions contenant au moins une substance éluée en même temps que le pF4, qui sont visées en tant que moyen (c) ci-dessus, on peut notamment mentionner les substances PBP, LA-pF4, βTG et leurs mélanges.

Parmi les complexes protéoglycan-pF4 visés en tant que moyen (G) ci-dessus, on peut notamment citer le complexe constitué par le protéoglycan dimère dans lequel chaque groupe protéoglycan est lié à quatre groupes pF4 tétramère et ayant un poids moléculaire moyen de 368 000 daltons, ledit complexe étant également dénommé "forme native du pF4" et présentant une forte affinité pour l'héparine. Ledit complexe peut être représenté par la formule suivante

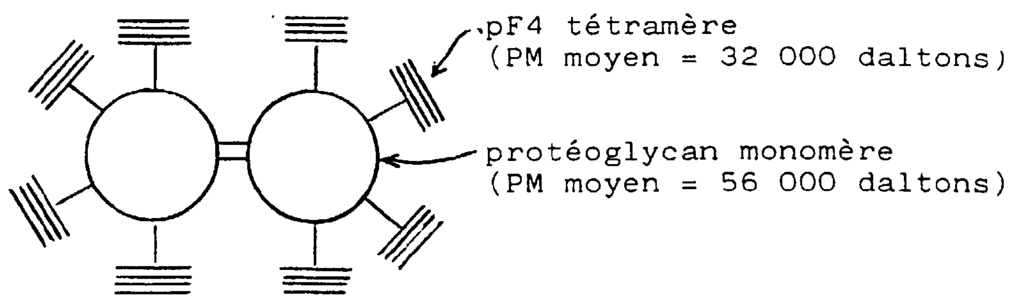


Les substances antigéniques Ag particulièrement préférées pour la détermination des thrombopénies induites par l'héparine sont choisies parmi l'ensemble constitué par

- le pF4 monomère ayant un poids moléculaire moyen de 8 000 daltons,
- le pF4 polymère, notamment le pF4 tétramère ayant un poids moléculaire moyen de 32 000 daltons,
- le protéoglycan ayant un poids moléculaire moyen de 56 000 daltons,
- les complexes protéoglycan-pF4, notamment le complexe constitué par le protéoglycan dimère dans lequel chaque groupe protéoglycan est lié à 4 groupes pF4 tétramère et ayant un poids moléculaire moyen de 368 000 daltons, et
- leurs mélanges.

Selon le meilleur mode de mise en oeuvre de l'invention, on préfère avantageusement utiliser des substances antigéniques $Ag_1$ qui sont des complexes desdites substances Ag sus-visées avec Hep.

Dans le cadre de la détermination des thrombopénies induites par l'héparine, la mise en oeuvre de la réaction précitée comprend :

1°) la formation et/ou l'activation de la substance antigénique selon le mécanisme :

$$Ag + Hep \rightarrow Ag\text{-}Hep \quad (2)$$

2°) l'une des réactions correspondantes :

$$Ag\text{-}Hep + anti(Hep) \rightarrow Ag\text{-}Hep\text{-}anti(Hep) \quad (3)$$

$$Ag\text{-}Hep + anti(Hep\text{-}Ag) \rightarrow Ag\text{-}Hep\text{-}anti(Hep\text{-}Ag) \quad (4)$$

$$Ag\text{-}Hep + anti(Hep\text{-}Ag\text{-}plaquette) \rightarrow Ag\text{-}Hep\text{-}anti(Hep\text{-}Ag\text{-}plaquette) \quad (5)$$

où

Hep est défini comme indiqué ci-dessus, et

Ag est pF4, un de ses polymères, le protéoglycan, un complexe protéoglycan-pF4 ou un de leurs mélanges.

Pour l'obtention des moyens (A)-(H) sus-visés, on procède au clivage ou à la lyse de plaquettes sanguines. De façon avantageuse, le pF4, ses polymères et les complexes protéoglycan-pF4 sont recueillis à partir de plaquettes sanguines lysées, par fixation sur gel d'héparine-agarose puis élution dudit gel à une température de 15-25° C, au moyen d'un éluant aqueux ayant, à un pH de 6,5-7,5, une force ionique supérieure ou égale à 0,60.

Selon l'invention, la formation du complexe résultant de la réaction de ladite substance antigénique avec ledit matériel anticorps est détectée par une méthode choisie parmi l'ensemble constitué par les méthodes EIA, RIA, FIA et agglutination.

Pour la révélation de la réaction précitée ou, dans le cas spécifique de la détermination des thrombopénies induites par l'héparine, des réactions (3)-(5), on peut mettre en oeuvre une méthode EIA dite de compétition ou encore une méthode EIA dite sandwich, selon un mécanisme qui comprend la réaction de ladite substance antigénique (1) avec ledit matériau anticorps (2), puis la réaction du complexe ainsi formé avec un anticorps anti(Ig) marqué, notamment un anticorps anti(IgA) marqué, anticorps anti(IgG) marqué ou anticorps anti(IgM) marqué.

A titre d'exemple, pour la détermination des thrombopénies induites par l'héparine, l'on fera réagir selon la méthode sandwich le produit des réactions (3)-(5), à savoir Ag-Hep-anti(Hep), Ag-Hep-anti(Hep-Ag) ou Ag-Hep-anti(Hep-Ag-plaquette), avec un anticorps de formule anti(Ig)-R*, où le moyen de marquage R* comprend un enzyme détectable par un substrat approprié; ainsi R* peut être POD et le substrat approprié correspondant peut être OPD.

A titre d'exemple pour la détermination des thrombopénies induites par Hep, l'on fera réagir selon la méthode dite de compétition la substance antigénique Ag ou $Ag_1$ selon l'invention (avantageusement fixée sur un support approprié) avec un mélange constitué par l'anticorps anti(Hep-Ag) provenant du plasma à étudier et un anticorps anti(Hep-Ag)-R*, où le moyen de marquage R* comprend un enzyme détectable par un substrat approprié, R* pouvant être POD et le substrat approprié correspondant pouvant être OPD comme indiqué ci-dessus.

Egalement à titre d'exemple pour la détermination des thrombopénies induites par Hep, l'on pourra utiliser une méthode d'agglutination selon laquelle l'on fait réagir la substance antigénique Ag ou $Ag_1$ fixée sur un support approprié (particules de latex, liposome, fragment de liposome, bentonite, charbon, or colloidal ou toutes autres particules inertes connues en immunochimie) avec le plasma du malade contenant l'anticorps anti(Hep-Ag). L'agglutination peut être mesurée par photométrie ou lue directement sur lame ou en tube.

En variante, le complexe antigène/anticorps ainsi obtenu, dans lequel l'antigène est fixé sur un support approprié (de préférence une paroi), pourra être révélé par réaction avec un anticorps marqué convenable (anticorps marqué par un enzyme, un moyen fluorogène, un radio-isotope, une particule de latex colorée, de l'or colloïdal, etc...).

D'un point de vue pratique, le milieu de dilution utilisé pour la mise en oeuvre de la réaction et sa révélation, quand on mesure au moyen d'un photomètre la variation de densité optique, est un milieu aqueux ayant, pour un plasma normal, une densité optique inférieure ou égale à 0,2 et mieux inférieure ou égale à 0,1. De préférence, une telle densité optique initiale est obtenue avec un milieu de dilution du plasma qui est un tampon phosphate contenant du sérum de chèvre, les conditions utilisées étant une dilution du plasma de sujet normal et du plasma pathologique à étudier de 1/50 à 1/100 (v/v) et une quantité de sérum de chèvre de 5 à 10 % en volume par rapport audit milieu de dilution. En d'autres termes, pour une détermination selon une méthode photométrique, l'on fera appel à un milieu de

dilution pour le plasma à étudier qui a une densité optique inférieure ou égale à 0,2 et mieux inférieure ou égale à 0,1.

Avec les milieux de dilution classiques, on obtient dans les mêmes conditions une densité optique pour le plasma normal supérieure ou égale à 0,6 qui ne permet pas d'apprécier correctement l'augmentation de la variation de densité optique lors de l'analyse relative aux plasmas pathologiques.

Les anticorps anti(Ig) et anti(Hep-Ag) qui interviennent dans la mise en oeuvre de l'invention peuvent être des anticorps polyclonaux ou monoclonaux. Quand on utilise des anticorps monoclonaux, on peut (si on le souhaite mais cela n'est pas essentiellement nécessaire dans la pratique de la présente invention) faire appel à leurs fragments F (ab) ou $F(ab')_2$ pour éviter toute interaction possible avec FR dès lors que ledit FR est très sensible vis-à-vis des fragments Fc.

Selon l'invention, on propose enfin un nécessaire pour la détermination des thrombopénies induites par l'héparine, caractérisé en ce qu'il comprend :

(1) au moins un échantillon de ladite substance antigénique Ag choisie parmi l'ensemble constitué par :

- le pF4 monomère ayant un poids moléculaire moyen de 8 000 daltons,
- le pF4 polymère, notamment le pF4 tétramère ayant un poids moléculaire moyen de 32 000 daltons,
- le protéoglycan ayant un poids moléculaire moyen de 56 000 daltons,
- le complexe protéoglycan-pF4 constitué par le protéoglycan dimère dans lequel chaque groupe protéoglycan est lié à 4 groupes pF4 trétramère, ledit complexe ayant un poids moléculaire moyen de 368 000 daltons,

ou au moins un échantillon d'un complexe Hep-Ag de ladite substance antigénique Ag avec Hep,

(2) au moins un anticorps anti(Ig) selon la revendication 13 ou un anticorps anti(Ag) selon la revendication 15, et le cas échéant, des milieux de dilution et d'autres réactifs.

D'autres avantages et caractéristiques de l'invention seront mieux compris à la lecture des exemples de réalisation qui suivent. L'ensemble de ces éléments n'est nullement limitatif mais est donné à titre d'illustration.

**EXEMPLE 1**

Obtention de pF4

On part d'un lysat plaquettaire congelé qu'on lave et décongèle trois fois de suite. Le lysat ainsi traité est mis en contact avec une solution aqueuse contenant du sulfate d'ammonium (60 % p/v); il se forme un précipité. On recueille le surnageant et on le soumet à une opération de dialyse. Le dialysat est déposé sur une colonne de gel d'héparine-agarose puis élué à RT au moyen d'un éluant aqueux (gradient de sel) ayant, à un pH de 6,5-7,5, une force ionique supérieure ou égale à 0,60.

L'élution avec un gradient de sel, à l'aide d'un analyseur-séparateur automatique, fournit un chromatogramme dans le système OD (en ordonnées) à 280 nm/volume élué exprimé en ml (en abscisses) dans lequel

a désigne le volume "mort";
b désigne un premier pic pour 30 ml (injection du gradient de sel);
c désigne une première fraction de 60 à 90 ml relative à des glycoprotéines et des traces de thrombospondine, présentant un pic maximal pour 70 ml;
d désigne une seconde fraction de 90 à 132 ml relative principalement à la thrombospondine, présentant un pic maximal pour 100 ml (thrombospondine + glycoprotéine) et un pic maximal pour 112 ml (thrombospondine);
e une troisième fraction de 132 à 180 ml relative principalement à βTG comprenant un pic maximal pour 142 ml (glycoprotéine) et un pic maximal pour 170 ml (βTG), et
f une quatrième fraction relative au pF4.

Les fractions des pics 70 (i.e. 70 ml), 100, 112, 142, 170 et pF4 ont été testées avec le surnageant avant dépôt et le volume mort après revêtement ("coating") sur les parois ou fonds de microcuvettes, vis-à-vis de plasmas pathologiques de sujets atteints de thrombopénies bien caractérisées induites par l'héparine, l'héparinate de calcium ou l'héparinate de magnésium et de plasmas de sujets normaux, lesdits plasmas étant dilués au 1/50è-1/100è au moyen d'un milieu de dilution (tampon phosphate additionné de 10 % v/v de sérum de chèvre).

Un immunoconjugué, un anticorps anti(Ig) marqué à la peroxydase [notamment anti(IgA)-POD, anti(IgG)-POD et anti(IgM)-POD]; a été utilisé pour la révélation (technique EIA sandwich) puis le développement de la coloration a été effectué au moyen du couple OPD/$H_2O_2$.

La réactivité comparée des plasmas normaux et des plasmas pathologiques a mis en évidence que la fraction

contenant le pF4 était la plus efficace pour fixer l'auto-anticorps anti(Y) responsable de la thrombopénie.

**EXEMPLE 2**

Détermination des thrombopénies induites par l'héparine

On préconise le protocole suivant pour la détermination des thrombopénies induites par l'héparine et ses sels métalliques.

On procède directement sur plasma selon une technique EIA sandwich. Les auto-anticorps générés par Hep (désignant ici l'héparine et/ou ses sels métalliques) et contenus dans le plasma, sont fixés par l'antigène d'origine plaquettaire en présence d'héparine (pF4-Hep) et révélés par un anticorps anti(Ig humaine)-POD [notamment anti(IgA)-POD, anti(IgG)-POD ou anti(IgM)-POD] puis développement de la coloration au moyen du couple OPD/$H_2O_2$.

Réactifs

Le nécessaire, kit ou trousse de dosage comprend les réactifs suivants :

- plaque préenduite ("precoated") : plaque sécable en 6 blocs ("ships") de 16 cupules chacun, revêtue par l'antigène pF4 en présence d'Hep, selon une proportion de 2 à 8 µg/ml (de préférence 5 µg/ml) de pF4 pour 0,02 à 1 UI/ml (de préférence 0,1 UI/ml) de Hep;
- anti(Ig) marqué : immunoglobulines de chèvre anti(IgA, G ou M humaines) couplées à la peroxydase [à savoir : anti(IgA)-POD, anti(IgG)-POD ou anti(IgM)-POD];
- milieu de dilution : tampon phosphate contenant 10 % (v/v) de sérum de chèvre;
- solution de lavage : solution aqueuse contenant un agent tensioactif TWEEN$^R$20, 20 fois concentré (à diluer au moment de l'emploi);
- substrat OPD : 5 à 10 comprimés renfermant chacun 2 mg de OPD; et
- le cas échéant, des échantillons de référence standardisés pour étalonnage, et une source de $H_2O_2$.

Principes opératoires

- Les plasmas à tester, qu'ils soient pathologiques ou normaux sont recueillis et traités comme suit : prélèvement du plasma sur citrate trisodique 0,109 M, centrifugation pendant 10 minutes à 3000 tours/minute et récupération du surnageant.
- L'échantillon de plasma à tester doit être dilué au 1/50è-1/100è au moyen du milieu de dilution.

Procédure

1. "Coating"

   La fixation de la substance antigénique pF4-Hep est réalisée dans chaque cupule au moyen de 5 µg/ml de pF4 et de 0,1 UI/ml d'Hep.
2. Addition de l'anticorps

   On ajoute 200 µl du milieu de dilution contenant le plasma à tester (dans lequel est présent le matériau auto-anticorps responsable de la thrombopénie induite par Hep) par cupule.
3. Incubation/lavage

   On incube pendant 2 h à RT, puis procède à 3 lavages successifs au moyen de la solution de lavage après avoir diluée celle-ci.
4. Addition de l'immunoconjugué

   On introduit dans chaque cupule 200 µl d'immunoconjugué marqué à la peroxydase.
5. Incubation/lavage

   On incube le milieu réactionnel résultant pendant 2 h à RT, puis procède à 3 lavages successifs au moyen de la solution de lavage diluée.
6. Coloration

   On ajoute 200 µl du couple OPD/$H_2O_2$ par cupule; incube pendant 3 minutes à RT; stoppe la réaction par addition de 50µl de $H_2SO_4$ 3M par cupule; et lit la valeur de OD à 492 nm.

**EXEMPLE 3**

Corrélation

Des essais cliniques ont été réalisés en parallèle selon le protocole de l'exemple 2 par comparaison avec la technique antérieure faisant appel à l'agglutination des plaquettes sanguines par l'héparine. Dans cette optique, le plasma de chaque patient hospitalisé présumé atteint de thrombopénie induite par l'hépa rine, a été séparé en deux lots, un pour chaque technique. Les résultats obtenus sont consignés dans le tableau I ci-après.

Ces résultats mettent en évidence que

(i) quand le test d'agrégation des plaquettes à l'héparine est douteux, la détermination selon l'invention permet de lever le doute : thrombopénie pour les patients No 4 et 15, absence de thrombopénie pour les patients No 2 et 25;
(ii) la détermination selon l'invention permet de pallier les erreurs de diagnostic du test d'agrégation pour les patients No14 et 16 (qui n'avaient manifestement pas de thrombopénie induite par l'héparine) et les patients No 17 et 22 (qui étaient manifestement atteints de thrombopénie induite par l'héparine); et
(iii) il existe une bonne corrélation pour les résultats obtenus pour le reste des autres patients.

Ces essais cliniques mettent en évidence l'intérêt de la détermination des thrombopénies selon l'invention, en ce qui concerne la rapidité et la fiabilité, par rapport à la technique antérieure d'agrégation des plaquettes.

**EXEMPLE 4**

Autres essais cliniques

Une seconde série d'essais cliniques a été réalisée selon le protocole de l'exemple 2 sur un lot de plasmas de patients atteints de thrombopénie induite par l'héparine par rapport à un lot de plasma de sujets normaux. Dans cette seconde série d'essais on a utilisé les plasmas de 21 patients thrombopéniques et de 32 sujets normaux, avec comme substance antigène un complexe pF4-héparine élaboré à partir des proportions suivantes : 5 µg/ml de pF4 et 0,1 UI/ml d'héparinate de calcium.

Les résultats obtenus avec des dilutions au 1/50è et au 1/100è des plasmas à tester, montrent l'intérêt de l'addition de 10 % v/v de sérum de chèvre dans le milieu de dilution pour avoir une OD toujours inférieure à 0,1 pour les plasmas normaux. Lorsque l'OD des plasmas normaux dilués au 1/50è ou au 1/100è est supérieure à 0,3 voire 0,6, il devient difficile d'apprécier les OD des plasmas thrombopéniques avec un photomètre.

**TABLEAU I**

| Patient | Détermination selon l'invention (a) | (b) | test d'agrégation à l'héparine (c) |
|---|---|---|---|
| 1 | 0,65 | 0,45 | A |
| 2 | 0,20 | 0,10 | B |
| 3 | 0,11 | 0,09 | C |
| 4 | 0,24 | 0,15 | B |
| 5 | 0,81 | 0,52 | A |
| 6 | 0,59 | 0,34 | A |
| 7 | 0,88 | 0,60 | A |
| 8 | 0,60 | 0,39 | A |
| 9 | 0,73 | 0,74 | A |
| 10 | 1,58 | 0,93 | A |
| 11 | 1,65 | 1,12 | A |
| 12 | 1,17 | 1,03 | A |
| 13 | 0,79 | 0,60 | A |
| 14 | 0,17 | 0,10 | A |
| 15 | 0,70 | 0,32 | B |
| 16 | 0,20 | 0,13 | A (d) |
| 17 | 0,67 | 0,44 | C |
| 18 | 0,80 | 0,53 | A |
| 19 | 0,57 | 0,46 | A |
| 20 | 0,15 | 0,10 | C |
| 21 | 0,72 | 0,38 | A |
| 22 | 0,62 | 0,42 | C |
| 23 | 0,51 | 0,31 | A |

**TABLEAU I (fin)**

| Patient | Détermination selon l'invention (a) | (b) | test d'agrégation à l'héparine (c) |
|---|---|---|---|
| 24 | 1,64 | 1,30 | A |
| 25 | 0,13 | 0,10 | B |
| Contrôle (e) | 0,12 (0,06-0,21) | 0,08 (0,04-0,13) | - |

Notes

(a) avec plasma dilué au 1/50è;
(b) avec plasma dilué au 1/100è;
(c) avec la notation suivante :
A résultat positif;
B résultat douteux;
C résultat négatif;
(d) résultat évalué à tort positif (erreur due à la grossesse de la patiente);
(e) effectué sur plasma de sujet normal valeur moyenne et intervalle.

## Revendications

1. Procédé pour la détermination des thrombopénies induites par une substance héparinique (Hep) en tant que drogue inductrice, ledit procédé étant caractérisé en ce qu'il comprend les étapes consistant :

(1°) à faire réagir (a) une substance antigénique (Ag ou $Ag_1$), la substance antigénique Ag étant choisie parmi

l'ensemble constitué par

(A) le facteur plaquettaire 4 (pF4),
(B) les fractions contenant le pF4,
(C) les fractions contenant au moins une substance éluée en même temps que le pF4,
(D) le pF4 recombinant et ses variants,
(E) les peptides synthétiques reprenant tout ou une partie de la séquence des aminoacides du pF4,
(F) le protéoglycan,
(G) les complexes protéoglycan-pF4, et
(H) leurs mélanges,

et la substance antigénique $Ag_1$ étant un complexe Hep-Ag de ladite substance héparinique Hep avec ladite substance antigénique Ag, avec (b) le plasma d'un patient susceptible de contenir un matériau anticorps anti (Hep-Ag), qui est généré dans l'organisme après administration de Hep, en cas de thrombopénie héparinique, et qui reconnaît Ag, selon la réaction

$$\text{Ag} + \text{anti(Hep-Ag)} \rightarrow \text{Ag-anti(Hep-Ag)},$$

ou

$$\text{(Hep-Ag)} + \text{anti(Hep-Ag)} \rightarrow \text{(Hep-Ag)-anti(Hep-Ag)} ;$$

puis,
(2°) à révéler le complexe produit résultant de ladite réaction.

2. Procédé suivant la revendication 1, caractérisé en ce que la substance héparinique Hep est choisie parmi l'ensemble constitué par

- l'héparine proprement dite ayant un PM moyen de 6 000-30 000 daltons et un pouvoir rotatoire $[\alpha]_D^{20}$ d'environ + 55° ;
- les dérivés et les fragments de ladite héparine ;
- les analogues de ladite héparine
- les substances contenant ladite héparine, l'un de ses dérivés ou l'un de ses analogues ; et
- leurs mélanges.

3. Procédé suivant la revendication 2, caractérisé en ce que lesdits dérivés sont choisis parmi les héparinates métalliques.

4. Procédé suivant la revendication 3, caractérisé en ce que lesdits héparinates métalliques sont choisis parmi les héparinates de calcium, de magnésium, de lithium et de sodium.

5. Procédé suivant la revendication 2, caractérisé en ce que lesdits analogues sont choisis parmi les héparinoïdes.

6. Procédé suivant la revendication 5, caractérisé en ce que lesdits héparinoïdes sont choisis parmi l'héparamine et ses sels, les chondroïtines et leurs sels et les héparines ayant un poids moléculaire moyen inférieur à 6 000 daltons.

7. Procédé suivant la revendication 1, caractérisé en ce que ladite substance antigénique Ag est constituée par ou contient le pF4, et a été recueillie, à partir de plaquettes sanguines lysées, par fixation sur gel d'héparine-agarose puis élution dudit gel à une température de 15-25° C, au moyen d'un éluant aqueux ayant, à un pH de 6,5-7,5, une force ionique supérieure ou égale à 0,60.

8. Procédé suivant la revendication 1, caractérisé en ce que la substance Ag, éluée en même temps que le pF4 selon l'élution de la revendication 7, est choisie parmi la protéine basique de plaquette (PBP) le pF4 de faible affinité (LA-pF4), la bêta-thromboglobuline (βTG) et leurs mélanges.

9. Procédé suivant la revendication 1 pour la détermination des thrombopénies induites par Hep, ledit procédé étant

caractérisé en ce que la substance antigénique $Ag_1$ est choisie parmi l'ensemble constitué par les complexes :

- du facteur plaquettaire 4 (pF4),
- des fractions contenant le pF4,
- des fractions contenant au moins une substance éluée en même temps que le pF4,
- du protéoglycan, et
- des complexes protéoglycan-pF4,

avec Hep tel que défini ci-dessus, et leurs mélanges.

**10.** Procédé suivant la revendication 8 ou 9, caractérisé en ce que ladite substance antigénique est choisie parmi l'ensemble constitué par :

- le pF4 monomère ayant un poids moléculaire moyen de 8 000 daltons,
- le pF4 polymère, notamment le pF4 tétramère ayant un poids moléculaire moyen de 32 000 daltons,
- le protéoglycan ayant un poids moléculaire moyen de 56 000 daltons,
- le complexe protéoglycan-pF4 constitué par le protéoglycan dimère dans lequel chaque groupe protéoglycan est lié à 4 groupes pF4 tétramère, ledit complexe ayant un poids moléculaire moyen de 368 000 daltons,
- les complexes de ces produits avec Hep, où Hep est défini comme indiqué ci-dessus ; et, leurs mélanges.

**11.** Procédé suivant la revendication 8 ou 9, caractérisé en ce que ladite substance antigénique Ag est le pF4 monomère ayant un poids moléculaire moyen de 8 000 daltons.

**12.** Procédé suivant la revendication 1, caractérisé en ce que la formation du complexe résultant de la réaction de ladite substance antigénique Ag ou $Ag_1$ avec ledit matériau anticorps anti(Hep-Ag) est détectée par une méthode choisie parmi l'ensemble constitué par les méthodes EIA, RIA, FIA et agglutination.

**13.** Procédé suivant la revendication 12, caractérisé en ce qu'il comprend la réaction de ladite substance antigénique avec ledit matériau anticorps pour l'obtention d'un complexe substance antigénique-matériau anticorps, puis la réaction du complexe, ainsi formé, avec un anticorps anti(Ig) marqué.

**14.** Procédé suivant la revendication 13, caractérisé en ce que l'anticorps anti(Ig) marqué est choisi parmi l'ensemble constitué par les anticorps anti(IgA), anti(IgG) et anti(IgM) marqués à la peroxydase.

**15.** Procédé suivant la revendication 12, caractérisé en ce qu'il comprend la réaction de ladite substance antigénique avec un mélange contenant ledit matériau anticorps et un anticorps anti(substance antigénique) marqué.

**16.** Procédé suivant l'une quelconque des revendications 1 et 12 à 15 pour la détermination selon une méthode photométrique des thrombopénies induites par Hep, caractérisé en ce que le plasma à tester est dilué, le milieu de dilution utilisé étant tel qu'il présente, pour un plasma de sujet normal à une concentration de 1/50-1/100 (v/v) dans ledit milieu, une densité optique inférieure ou égale à 0,2 et mieux inférieure ou égale à 0,1.

**17.** Procédé suivant la revendication 16, caractérisé en ce que ledit milieu de dilution du plasma est un tampon phosphate contenant du sérum de chèvre.

**18.** Procédé suivant l'une quelconque des revendications 1 et 2, caractérisé en ce que

Ag est le pF4, et
Hep est l'héparine ayant un poids moléculaire moyen de 6 000-30 000 daltons et un pouvoir rotatoire $[\alpha]_D^{20}$ d'environ + 55°.

**19.** Procédé suivant la revendication 1, caractérisé en ce que ladite substance antigénique Ag est mise en réaction avec le matériau anticorps anti(Hep-Ag) en présence de Hep.

**20.** Nécessaire pour la détermination des thrombopénies induites par Hep, caractérisé en ce qu'il comprend

(1) au moins un échantillon de ladite substance antigénique Ag choisie parmi l'ensemble constitué par :

- le pF4 monomère ayant un poids moléculaire moyen de 8 000 daltons,
- le pF4 polymère, notamment le pF4 trétramère ayant un poids moléculaire moyen de 32 000 daltons,
- le protéoglycan ayant un poids moléculaire moyen de 56 000 daltons,
- le complexe protéoglycan-pF4 constitué par le protéoglycan dimère dans lequel chaque groupe protéoglycan est lié à 4 groupes pF4 tétramère, ledit complexe ayant un poids moléculaire moyen de 368 000 daltons,

ou au moins un échantillon d'un complexe Hep-Ag de ladite substance antigénique Ag avec Hep,
(2) au moins un anticorps anti(Ig) selon la revendication 13 ou un anticorps anti(Ag) selon la revendication 15, et,

le cas échéant, des milieux de dilution et d'autres réactifs.

## Patentansprüche

**1.** Verfahren zum Nachweis von Thrombopenien, induziert durch eine heparinartige Substanz (Hep) als induzierender Arzneistoff, wobei das Verfahren dadurch gekennzeichnet ist, daß es die folgenden Schritte umfaßt:

(1) Reagierenlassen (a) einer antigenen Substanz (Ag oder $Ag_1$), wobei die antigene Substanz Ag ausgewählt ist aus der Gruppe bestehend aus:

(A) dem Plättchenfaktor 4 (pF4);
(B) den Fraktionen, die pF4 enthalten;
(C) den Fraktionen, die mindestens eine Substanz enthalten, die zur gleichen Zeit wie pF4 eluiert wird;
(D) dem rekombinanten pF4 und seinen Varianten;
(E) den synthetischen Peptiden, die die Gesamtheit oder einen Teil der Aminosäuresequenz von pF4 aufweisen;
(F) dem Proteoglycan;
(G) den Proteoglycan-pF4-Komplexen; und
(H) ihren Gemischen;

und wobei die antigene Substanz $Ag_1$ ein Komplex Hep-Ag aus der heparinartigen Substanz Hep mit der antigenen Substanz Ag ist;
mit (b) dem Plasma eines Patienten, der möglicherweise ein Antikörpermaterial anti-(Hep-Ag) besitzt, das im Fall von npariner Thrombopenie in dem Organismus nach Verabreichung des Hep erzeugt wurde und das Ag gemäß der folgenden Reaktion erkennt

$$\text{Ag + anti-(Hep-Ag)} \rightarrow \text{Ag-anti-(Hep-Ag)},$$

oder

$$\text{(Hep-Ag) + anti-(Hep-Ag)} \rightarrow \text{(Hep-Ag)-anti-(Hep-Ag)};$$

und anschließend
(2) Nachweis des erzeugten Komplexes, der aus der Reaktion resultiert.

**2.** Verfahren nach Anspruch 1, dadurch gekennzeichnet, daß die heparinartige Substanz Hep ausgewählt ist aus der Gruppe bestehend aus:

- Heparin mit einem mittleren Molekulargewicht von 6 000 - 30 000 Dalton und einer spezifischen Drehung $[\alpha]_D^{20}$ von ca. +55°;
- den Derivaten und den Fragmenten dieses Heparins;
- den Analogen dieses Heparins;
- den Substanzen, die dieses Heparin, eines seiner Derivate oder eines seiner Analoge enthalten; und
- ihren Gemischen.

**3.** Verfahren nach Anspruch 2, dadurch gekennzeichnet, daß die Derivate ausgewählt sind aus metallischen Heparinaten.

**4.** Verfahren nach Anspruch 3, dadurch gekennzeichnet, daß die metallischen Heparinate ausgewählt sind aus Calcium-, Magnesium-, Lithium- und Natrium-Heparinaten.

**5.** Verfahren nach Anspruch 2, dadurch gekennzeichnet, daß die Analogen ausgewählt sind aus den Heparinoiden.

**6.** Verfahren nach Anspruch 5, dadurch gekennzeichnet, daß die Heparinoiden ausgewählt sind aus Heparamin und seinen Salzen, den Chondroitinen und ihren Salzen und den Heparinen mit einem mittleren Molekulargewicht von weniger als 6 000 Dalton.

**7.** Verfahren nach Anspruch 1, dadurch gekennzeichnet, daß die antigene Substanz Ag von pF4 gebildet wird oder pF4 enthält und gewonnen wurde ausgehend von lysierten Blutplättchen durch Fixierung auf einem Heparin-Agarose-Gel und anschließender Elution von dem Gel bei einer Temperatur von 15 bis 25°C mittels eines wäßrigen Elutionsmittels, das bei einem pH-Wert von 6,5 bis 7,5 eine Ionenstärke von 0,6 oder mehr hat.

**8.** Verfahren nach Anspruch 1, dadurch gekennzeichnet, daß die Substanz Ag, die bei der Elution nach Anspruch 7 zur gleichen Zeit wie pF4 eluiert wird, ausgewählt ist aus dem basischen Blutplättchen-Protein (PBP), dem pF4 mit geringer Affinität (LA-pF4), dem β-Thromboglobulin (β-TG) und deren Gemischen.

**9.** Verfahren nach Anspruch 1 zum Nachweis von durch Hep induzierten Thrombopenien, wobei das Verfahren dadurch gekennzeichnet ist, daß die antigene Substanz $Ag_1$ ausgewählt ist aus der Gruppe bestehend aus den Komplexen:

- des Plättchenfaktors 4 (pF4);
- der Fraktionen, die den pF4 enthalten;
- der Fraktionen, die mindestens eine Substanz enthalten, die zur gleichen Zeit wie pF4 eluiert wird;
- des Proteoglycans; und
- der Proteoglycan-pF4-Komplexe;

mit Hep wie oben definiert; und ihren Gemischen.

**10.** Verfahren nach Anspruch 8 oder 9, dadurch gekennzeichnet, daß die antigene Substanz ausgewählt ist aus der Gruppe bestehend aus:

- dem pF4-Monomer, das ein mittleres Molekulargewicht von 8 000 Dalton hat;
- dem pF4-Polymer, insbesondere dem pF4-Tetramer, das ein mittleres Molekulargewicht von 32 000 Dalton hat;
- dem Proteoglycan, das ein mittleres Molekulargewicht von 56 000 Dalton hat;
- dem Proteoglycan-pF4-Komplex, bestehend aus dem Proteoglycan-Dimer, in dem jeder Proteoglycanrest an 4 pF4-Tetramerreste gebunden ist, wobei der Komplex ein mittleres Molekulargewicht von 368 000 Dalton hat;
- den Komplexen dieser Produkte mit Hep, wobei Hep wie oben angegeben definiert ist; und ihren Gemischen.

**11.** Verfahren nach Anspruch 8 oder 9, dadurch gekennzeichnet, daß die antigene Substanz Ag das pF4-Monomer mit einem mittleren Molekulargewicht von 8 000 Dalton ist.

**12.** Verfahren nach Anspruch 1, dadurch gekennzeichnet, daß die aus der Reaktion der antigenen Substanz Ag oder $Ag_1$ mit dem Antikörpermaterial anti-(Hep-Ag) resultierende Erzeugung des Komplexes nachgewiesen wird mittels einer Methode ausgewählt aus der Gruppe bestehend aus den Methoden EIA, RIA, FIA und Agglutination.

**13.** Verfahren nach Anspruch 12, dadurch gekennzeichnet, daß es die Reaktion der antigenen Substanz mit dem Antikörpermaterial umfaßt, um einen antigene Substanz-Antikörper-Komplex zu erhalten, und anschließend die Reaktion des so gebildeten Komplexes mit einem markierten anti-(Ig)-Antikörper.

**14.** Verfahren nach Anspruch 13, dadurch gekennzeichnet, daß der markierte anti-(Ig)-Antikörper ausgewählt ist aus der Gruppe bestehend aus den mit Peroxidase markierten Antikörpern anti-(IgA), anti-(IgG) und anti-(IgM).

**15.** Verfahren nach Anspruch 12, dadurch gekennzeichnet, daß es die Reaktion der antigenen Substanz mit einem

Gemisch umfaßt, das das Antikörpermaterial und einen markierten anti-(antigene Substanz)-Antikörper enthält.

**16.** Verfahren nach einem der Ansprüche 1 und 12 bis 15 zum Nachweis von durch Hep induzierten Thrombopenien mittels einer photometrischen Methode, dadurch gekennzeichnet, daß das zu testende Plasma verdünnt wird, und das zur Verdünnung verwendete Mittel derart ist, daß es, bei einem Plasma eines Normalpatienten bei einer Konzentration von 1/50 - 1/100 (Vol./Vol.) in diesem Mittel, eine optische Dichte von 0,2 oder weniger aufweist und vorzugsweise von 0,1 oder weniger.

**17.** Verfahren nach Anspruch 16, dadurch gekennzeichnet, daß das Verdünnungsmittel für das Plasma ein Phosphatpuffer ist, der Ziegenserum enthält.

**18.** Verfahren nach einem der Ansprüche 1 und 2, dadurch gekennzeichnet, daß

Ag der pF4 ist; und
Hep Heparin mit einem mittleren Molekulargewicht von 6 000 - 30 000 Dalton und einer spezifischen Drehung $[\alpha]_D^{20}$ von ca. +55° ist.

**19.** Verfahren nach Anspruch 1, dadurch gekennzeichnet, daß die antigene Substanz Ag mit dem Antikörpermaterial anti-(Hep-Ag) in Gegenwart von Hep zur Reaktion gebracht wird.

**20.** Testbesteck zum Nachweis von durch Hep induzierten Thrombopenien, dadurch gekennzeichnet, daß es enthält:

(1) mindestens eine Probe der antigenen Substanz Ag ausgewählt aus der Gruppe bestehend aus

- dem pF4-Monomer mit einem mittleren Molekulargewicht von 8 000 Dalton;
- dem pF4-Polymer, insbesondere dem pF4-Tetramer mit einem mittleren Molekulargewicht von 32 000 Dalton;
- dem Proteoglycan mit einem mittleren Molekulargewicht von 56 000 Dalton;
- dem Proteoglycan-pF4-Komplex gebildet aus dem Proteoglycan-Dimer, in dem jeder Proteoglycanrest gebunden ist an 4 pF4-Tetramerreste, und wobei der Komplex ein mittleres Molekulargewicht von 368 000 Dalton hat;

oder mindestens eine Probe mit einem Hep-Ag-Komplex der antigenen Substanz Ag mit Hep;
(2) mindestens einen anti-(Ig)-Antikörper nach Anspruch 13 oder einen anti-(Ag)-Antikörper nach Anspruch 15; und

gegebenenfalls Verdünnungsmittel und andere Reagenzien.

## Claims

**1.** A method for the determination of thrombopenia induced by a heparin substance (Hep) as an inductor drug, said method comprising the steps consisting of

(1°) reacting (a) an antigenic substance (Ag or $Ag_1$), the antigenic substance being selected from the group consisting of

(A) platelet factor 4 (pF4),
(B) fractions containing pF4,
(C) fractions containing at least one substance eluted as the same time as pF4,
(D) recombinant pF4 and its variants,
(E) synthetic peptides reproducing all or part of the amino acid sequence of pF4,
(F) proteoglycan,
(G) proteoglycan-pF4 complexes, and
(H) mixtures thereof,

and the antigenic substance $Ag_1$ being a complex Hep-Ag of said heparin substance Hep with said antigenic substance Ag,

with (b) the plasma of a patient susceptible to contain an antibody anti(Hep-Ag), which is generated in the organism after administration of Hep, in case of heparin-induced thrombopenia, and which recognizes Ag according to the reaction

$$Ag + anti(Hep\text{-}Ag) \rightarrow Ag\text{-}anti(Hep\text{-}Ag),$$

or

$$(Hep\text{-}Ag) + anti(Hep\text{-}Ag) \rightarrow (Hep\text{-}Ag)\text{-}anti(Hep\text{-}Ag) \; ;$$

then,
(2°) revealing the complex product resulting from said reaction.

**2.** A method according to claim 1, wherein Hep is selected from the group consisting of

- heparin itself, having an average MW of 6000-30000 daltons and an optical rotation $[\alpha]_D^{20}$ of about +55° ;
- derivatives and fragments of said heparin ;
- analogs of said heparin ;
- substances containing said heparin one of its derivatives or one of its analogs ; and
- mixtures thereof.

**3.** A method according to claim 2, wherein said heparin derivatives are selected from the metal heparinates.

**4.** A method according to claim 3, wherein said metal heparinates are selected from the calcium, magnesium, lithium and sodium heparinates.

**5.** A method according to claim 2, wherein said heparin analogs are selected from the heparinoids.

**6.** A method according to claim 5, wherein said heparinoids are selected from the heparamine and its salts, chondroitins and their salts and heparins having an average molecular weight of less than 6000 daltons.

**7.** A method according to claim 1, wherein said antigenic substance Ag consists of or contains pF4 and has been collected form lyzed blood platelets by binding to heparin-agarose gel and then elution of said gel, at a temperature of 15-25°C, by means of an aqueous eluent having, at a pH of 6.5-7.5, an ionic strength greater than or equal to 0.60.

**8.** A method according to claim 1, wherein the substance Ag, which is eluted at the same time as pF4 as recited in claim 7, is selected from the platelet basic protein (PBP), low-affinity pFA (LA-pF4, beta-thromboglobulin (βTG) and mixtures thereof.

**9.** A method according to claim 1 for the determination of heparin-induced thrombopenia, in which method the antigenic substance $Ag_1$ is selected from the group consisting of complexes of

- platelet factor 4 (pF4),
- fractions containing pF4,
- fractions containing at least one substance eluted at the same time as pF4,
- proteoglycan,
- proteoglycan-pF4 complexes,

with Hep as defined above and mixtures thereof.

**10.** A method according to claim 8 or claim 9 wherein said antigenic substance is selected from the group consisting of

- monomeric pF4 having an average molecular weight of 8000 daltons,
- polymeric pF4, especially tetrameric pF4 having an average molecular weight of 32000 daltons,
- proteoglycan having an average molecular weight of 56000 daltons,
- the proteoglycan-pF4 complex consisting of dimeric proteoglycan in which each proteoglycan group is bonded

to 4 tetrameric pF4 groups, said complex having an average molecular weight of 368000 daltons, and

- complexes of these products with Hep, where Hep is defined as indicated above ; and,
- mixture thereof.

**11.** A method according to claim 8 or claim 9, wherein said antigenic substance Ag is monomeric pF4 having an average molecular weight of 8000 daltons.

**12.** A method according to claim 1, wherein the formation of the complex resulting from the reaction of said antigenic substance Ag or $Ag_1$ with said anti(Hep-Ag) antibody material is detected by a method selected from the group consisting of the EIA, RIA, FIA and agglutination methods.

**13.** A method according to claim 12, which comprises reacting said antigenic substance with said antibody material to give an antigenic substance-antibody material complex, and then reacting the complex thus formed with a labeled anti(Ig) antibody.

**14.** A method according to claim 13, wherein the labeled anti(Ig) antibody is selected from the group consisting of anti (IgA), anti(IgG) and anti(IgM) antibodies labeled with peroxidase.

**15.** A method according to claim 12, which comprises reacting said antigenic substance with a mixture containing said antibody material and a labeled anti(antigenic substance) antibody.

**16.** A method according to any one of claims 1 and 12 to 15 for the determination of heparin-induced thrombopenia by a photometric method, wherein the medium for diluting the plasma to be studied is such that it has an optical density less than or equal to 0.2 and preferably less than or equal to 0.1 for a normal subject's plasma at a concentration of 1/50-1/100 (v/v) in said medium.

**17.** A method according to claim 16 wherein said plasma diluting medium is a phosphate buffer containing goat's serum.

**18.** A method acording to any one of claims 1 and 2, wherein

Ag is pF4, and
Hep is heparin having an average MW of 6000-30000 daltons and an optical rotation $[\alpha]_D^{20}$ of about +55°.

**19.** A method according to claim 1, wherein said antigenic substance Ag is reacted with the anti(Hep-Ag) antibody material in the presence of Hep.

**20.** A kit for the determination of heparin-induced thrombopenia, which comprises

(1) at least one sample of said antigenic substance selected from the group consisting of

- monomeric pF4 having an average molecular weight of 8000 daltons,
- polymeric pF4, especially tetrameric pF4 having an average molecular weight of 32000 daltons,
- proteoglycan having an average molecular weight of 56000 daltons, and
- proteoglycan-pF4 complexes, especially the complex consisting of dimeric proteoglycan in which each proteoglycan group is bonded to 4 tetrameric pF4 groups, and having an average molecular weight of 368000 daltons,

or at least one sample of a Hep-Ag complex of said antigenic substance Ag with Hep,
(2) at least one anti(Ig) antibody according to claim 13 or one anti(Ag) antibody according to claim 15, and if appropriate, dilution media and other reagents.